Europäisches Patentamt

European Patent Office

Office européen des brevets

(19)

(11) Numéro de publication: **0 206 939**
**A1**

## (12) DEMANDE DE BREVET EUROPEEN

(21) Numéro de dépôt: **86401349.5**

(22) Date de dépôt: **19.06.86**

(51) Int. Cl.⁴: **C 12 N 15/00**, C 12 P 21/02, A 61 K 37/02, A 61 K 35/76, C 12 N 5/00, C 12 N 7/00

---

(30) Priorité: **21.06.85 FR 8509480**

(43) Date de publication de la demande: **30.12.86**
**Bulletin 86/52**

(84) Etats contractants désignés: **AT BE CH DE FR GB IT LI LU NL SE**

(71) Demandeur: **TRANSGENE S.A., 16, rue Henri Regnault, F-92400 Couirbevoie (FR)**

(72) Inventeur: **Kieny, Marie-Paul, 11, rue de Gascogne, F-67000 Strasbourg (FR)**
Inventeur: **Sondermeyer, Paul, 32, rue Raphael, F-67200 Strasbourg (FR)**
Inventeur: **Lecocq, Jean-Pierre, 6, rue du Champ du Feu, F-67460 Reichstett (FR)**

(74) Mandataire: **Warcoin, Jacques et al, Cabinet Régimbeau 26, avenue Kléber, F-75116 Paris (FR)**

---

(54) **Expression de l'il-2 humaine dans les cellules de mammifères par un poxvirus recombiné.**

(57) La présente invention concerne un poxvirus, caractérisé en ce qu'il comporte tout ou partie de la séquence d'ADN codant pour une protéine IL-2 humaine.

EXPRESSION DE L'IL-2 HUMAINE DANS LES CELLULES DE
MAMMIFERES PAR UN POXVIRUS RECOMBINE.

L'interleukine 2, IL-2, a été décrite, à l'origine, comme le facteur de croissance des lymphocytes-T. Cependant, de nombreuses expériences chez la souris ont mené à la conclusion que cette molécule joue en fait, un rôle essentiel dans l'induction de diverses réponses immunitaires (Morgan et al., 1976). Il a été montré que son administration in vivo augmentait la fonction "killer" naturelle, induisait des fonctions immunitaires chez les souris "nude",que son administration en conjonction avec des lymphocytes immuns avait un effet anti-tumoral (Hefenerder et al., 1983). In-vitro, l'IL-2 agit comme un facteur auxiliaire dans les réponses des cellules B et T, augmente la génération de lymphocytes-T cytotoxiques, maintient à long-terme des cultures de cellules cytotoxiques, et favorise le recouvrement de la fonction immunitaire de lymphocytes dans certains états immunodéficients. (Watson et Mochiguki, 1980).

L'IL-2 humaine est produite en quantité minime par les cellules -T "Helper" présentes dans les lymphocytes du sang périphérique. Cette sécrétion d'IL-2 peut être stimulée par incubation en présence d'antigènes tels que la phytohemagglutinine ou les esters de phorbol.

De plus, il existe une lignée de cellules -T de leucémie humaine, la lignée Jurkat, (Hansen et al., 1980) qui produit des quantités importantes d'IL-2 après un traitement mitogène.

Le clonage de la séquence de c-ADN codant pour l'IL-2 a été initialement publié par Taniguchi et al., (1983). Nous avons également cloné un c-ADN codant pour l'IL-2 humaine et obtenu un haut niveau d'expression dans E.coli et dans la levure.

Toutefois, la protéine produite par ces deux organismes sera différente de son équivalent naturel au niveau de son extrémité $NH_2$ qui peut contenir un résidu supplémentaire de formyl-méthionine. De plus, la protéine synthétisée par E.coli ne sera pas sécrétée et ne présentera aucune des modifications post-traductionnelles qui normalement se produisent dans les lymphocytes. Ceci est un argument en faveur du développement d'un système exprimant le c-ADN de l'IL-2 dans un environnement plus proche de son hôte naturel, c'est-à-dire dans une cellule de mammifère.

On décrit ci-après, un système basé sur un Poxvirus dans lequel est intégré le gène de l'IL-2, qui permet de synthétiser une protéine qui sera secrétée par divers types de cellules eucaryotes. De plus, comme le virus peut se propager chez des animaux vivants, on pourra réaliser des études in vivo de l'effet de l'IL-2 produite localement sur diverses réponses immunitaires.

Plusieurs groupes ont récemment mis en évidence l'utilisation de recombinants vivants du virus de la vaccine pour exprimer un antigène de l'Influenza, de l'Hépatite B et la glycoprotéine de la rage, pour immuniser contre ces maladies (Smith et al., 1983 ; Panicali et al., 1983 ; Kieny et al., 1984).

L'expression d'une séquence codante pour une protéine exogène dans le virus de la vaccine (VV) implique nécessairement deux étapes :
1) la séquence codante doit être alignée avec un promoteur de VV et être insérée dans un segment non essentiel de l'ADN de VV cloné dans un plasmide bactérien approprié;
2) les séquences d'ADN de VV situées de part et d'autre doivent permettre des recombinaisons homologues in vivo entre le plasmide et le génome viral. Une double recombinaison réciproque conduit à un transfert de l'insert d'ADN du plasmide dans le génome viral dans lequel il est propagé et exprimé (Panicali et Paoletti, 1982 ; Mackett et al., 1982 ; Smith et al., 1983 ; Panicali et al., 1983).

La présente invention concerne un Poxvirus caractérisé en ce qu'il comporte tout ou partie de la séquence d'ADN codant pour une protéine IL-2 humaine. Ce virus comportera de préférence des éléments assurant l'expression d'une IL-2 mature complète qui sera secrétée par les cellules dans le milieu. On entend désigner par "IL-2 mature complète" une IL-2 qui, in vitro, mais de préférence in vivo, présente des caractéristiques immunogéniques identiques ou voisines de l'IL-2 humaine vraie.

La présente invention concerne, de façon générale, l'utilisation des Poxvirus, mais sera décrite plus particulièrement à l'aide du représentant classique du genre orthopoxvirus, à savoir le virus de la vaccine.

Le virus comportera de préférence l'ensemble des éléments assurant l'expression de la protéine IL-2.

En particulier, il comportera un promoteur d'un gène du Poxvirus, par exemple celui du gène de la protéine de 7,5 K de la vaccine, noté P 7.5 K qui contrôlera l'expression de la séquence d'ADN codant pour l'IL-2, nommée ci-après séquence d'ADN (IL-2). Cet ensemble promoteur/séquence d'ADN (IL-2) sera inséré dans un gène du virus de la vaccine, par exemple le gène TK, ce qui fournit une possibilité de sélection comme cela sera expliqué ci-après.

La préparation du virus selon l'invention comporte notamment les étapes suivantes :

1) une restructuration du c-ADN par addition de la séquence codant pour le peptide-signal, séquence qui a été synthétisée *in vitro* ;

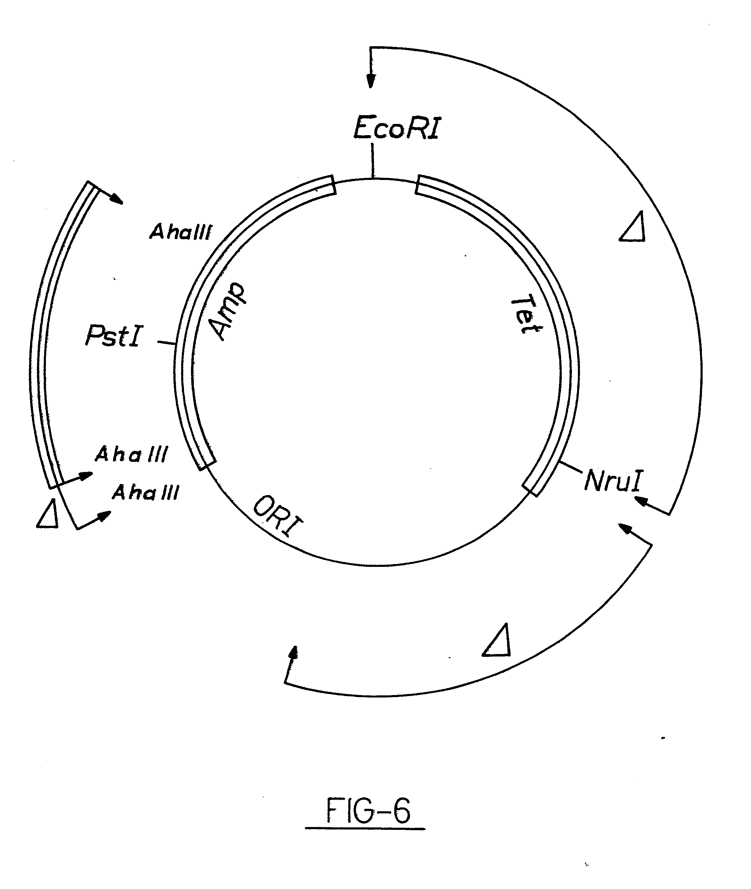

2) la synthèse d'un mini-plasmide pTG1H à partir de pBR322 (figure 6) ;

3) l'insertion dans ce mini-plasmide du fragment Hin-J portant le gène TK de VV ;

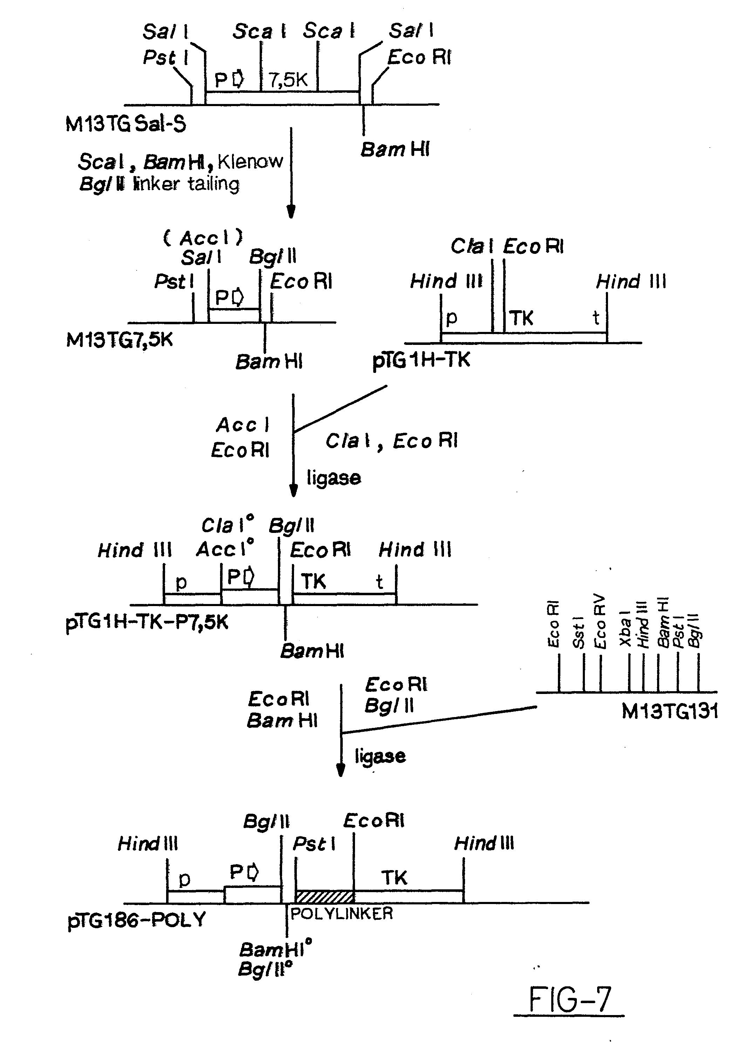

4) l'insertion dans le gène TK du promoteur de la protéine 7,5 K (figure 7) ;

5) l'insertion d'un polylinker en aval du promoteur P 7,5 K ;

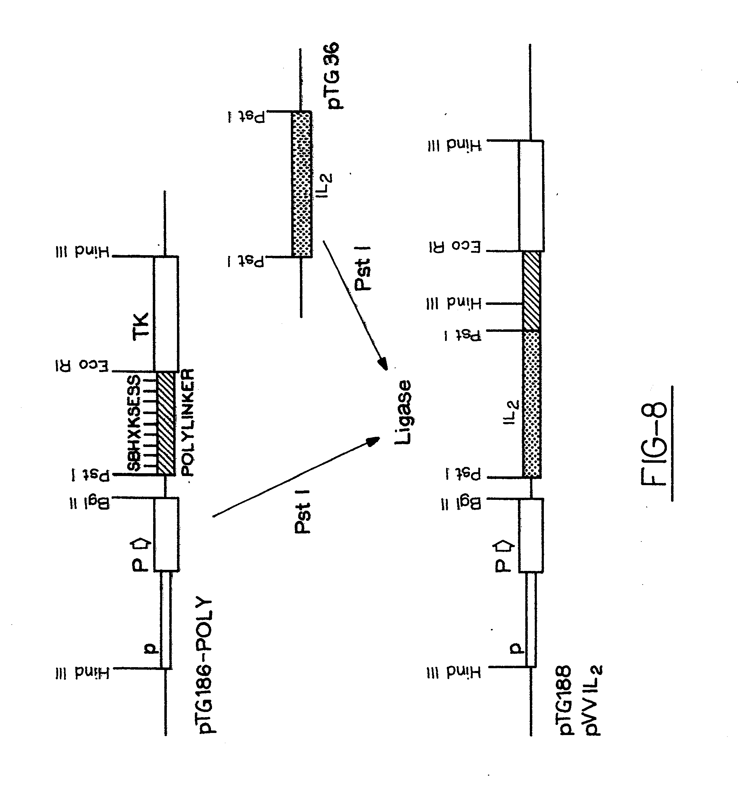

6) l'insertion dans un des sites de restriction du polylinker de la séquence c-ADN complète codant pour la protéine IL-2 (figure 8) ;

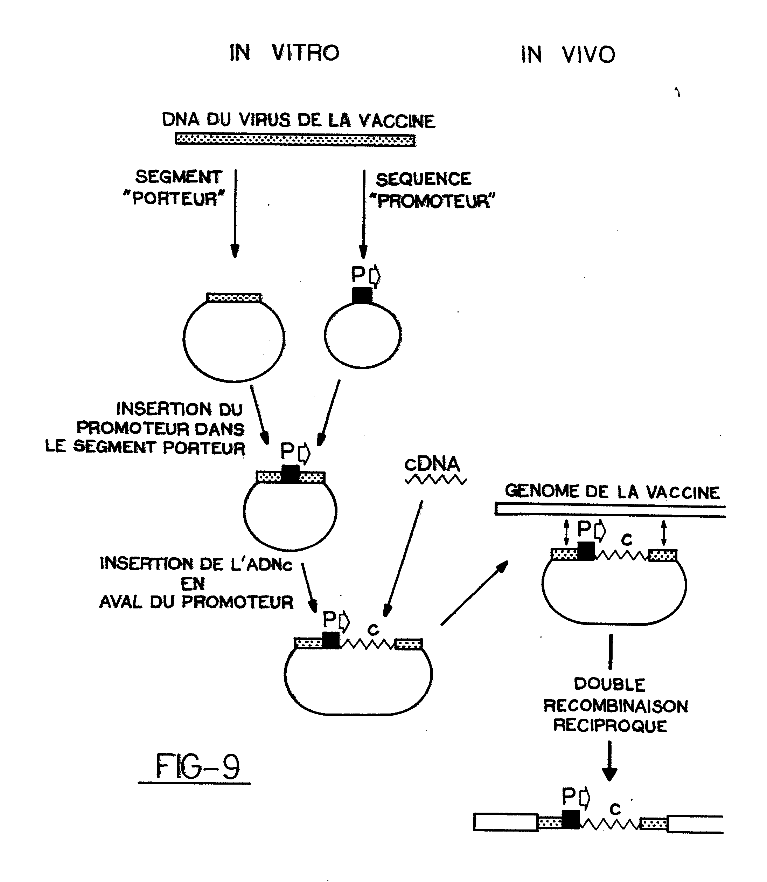

7) le clonage des éléments essentiels de ce dernier plasmide dans le virus de la vaccine (figure 9) ;

La présente invention concerne aussi les cellules eucaryotes transfectées par des virus recombinés IL-2, et en particulier les cellules BHK.

La présente invention concerne aussi le procédé de préparation de la protéine IL-2 obtenue par la culture des cellules eucaryotes transfectées.

La présente invention concerne aussi l'utilisation du virus recombiné vaccine-IL-2, par inoculation de ce virus vivant, à l'homme ou à des animaux, de manière à produire localement et transitoirement de l'IL-2, pendant que le virus se multiplie et jusqu'à ce que la réponse immunitaire de l'hôte arrête cette multiplication virale qui est la source de l'IL-2.

La présente invention concerne aussi une substance pharmaceutiquement acceptable jouant le rôle de support permettant l'administration du virus vivant par voie injectable.

La présente invention concerne aussi l'utilisation du virus recombiné vaccine-IL-2 vivant en combinaison avec un autre antigène vaccinal comme un virus ou une partie immunogène d'un virus pour stimuler localement la réponse immunitaire, notamment de type T, contre cet antigène.

La présente invention concerne enfin l'IL-2 humaine utilisée en tant qu'agent thérapeutique isolé ou combiné à d'autres principes actifs.

Les exemples suivants illustrent les propriétés des différents composants obtenus.

Les figures jointes à la description représentent successivement :

- 1 : la séquence du c-ADN de l'IL-2 portée par le pTG 26,

- 2 : la création d'un site de restriction (Sal I/Acc I) à l'extrémité 5' de la séquence du c-ADN portée par le pTG 26,

- 3 : la préparation du fragment de 92 paires de bases avec des oligonucléotides synthétiques,

- 4 : l'insertion de ce fragment entre les sites Bgl II et Sal I présents sur le pTG 28,

- 5 : la séquence du c-ADN portée par le pTG 36,

- 6 : le mini-plasmide pTG 1 H,

- 7 : l'insertion dans le gène TK du promoteur de la protéine 7,5 K,

- 8 : l'insertion dans un des sites de restriction du polylinker de la séquence c-ADN complète codant pour IL-2,

- 9 : le clonage des éléments essentiels du plasmide dans le virus de la vaccine,

-10 : l'analyse de l'ADN du virus recombiné VV-IL-2 par autoradiographie.

Les différents matériels mis en oeuvre sont identifiés dans les exemples.

Sauf indication contraire, les enzymes sont utilisés dans les conditions préconisées par le fabricant et les techniques mises en oeuvre sont connues de l'homme de métier.

Les séquences d'acides aminés et les séquences de nucléotides représentées sur les figures ne sont pas reprises dans le corps de la description pour ne pas l'alourdir mais elles en constituent une partie intégrante.

EXEMPLE 1 :

Restructuration de la séquence codant pour l'IL-2 :

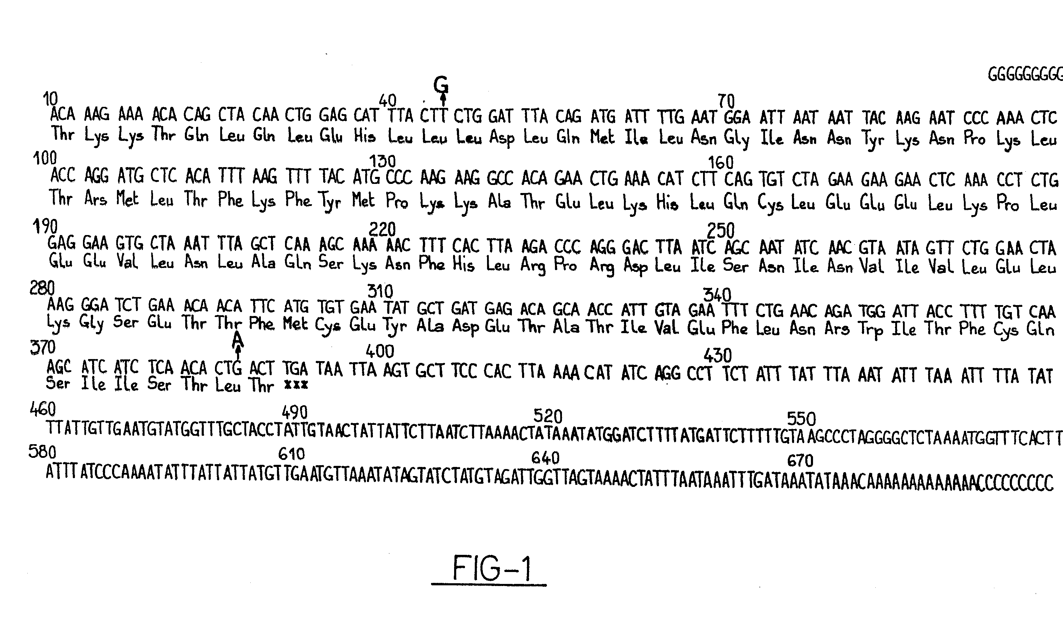

La figure 1 présente la séquence du c-ADN portée par le plasmide pTG 26, isolée à l'origine à partir d'une banque préparée avec de l'ARN provenant de lymphocytes stimulés par des mitogènes. Cette séquence diffère de celle publiée par TANIGUCHI (5) en positions 45 (G $\longrightarrow$ T) et 387 (G $\rightarrow$ A) mais ces différences sont silencieuses au niveau de la protéine.

D'autre part, une région de 80 paires de bases à l'extrémité codant pour le peptide signal et pour les 6 premiers acides aminés de la protéine mature est absente.

Cette partie de la séquence est probablement essentielle pour l'expression de la protéine IL-2 dans les cellules eucaryotes et pour sa sécrétion dans le milieu.

Cette séquence doit donc être synthétisée et intégrée en amont de la séquence portée par le pTG 26.

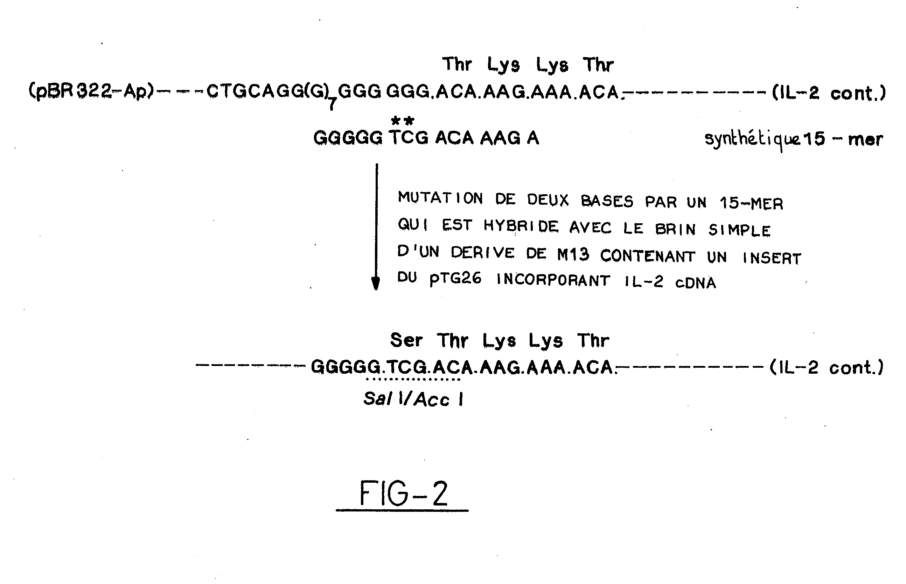

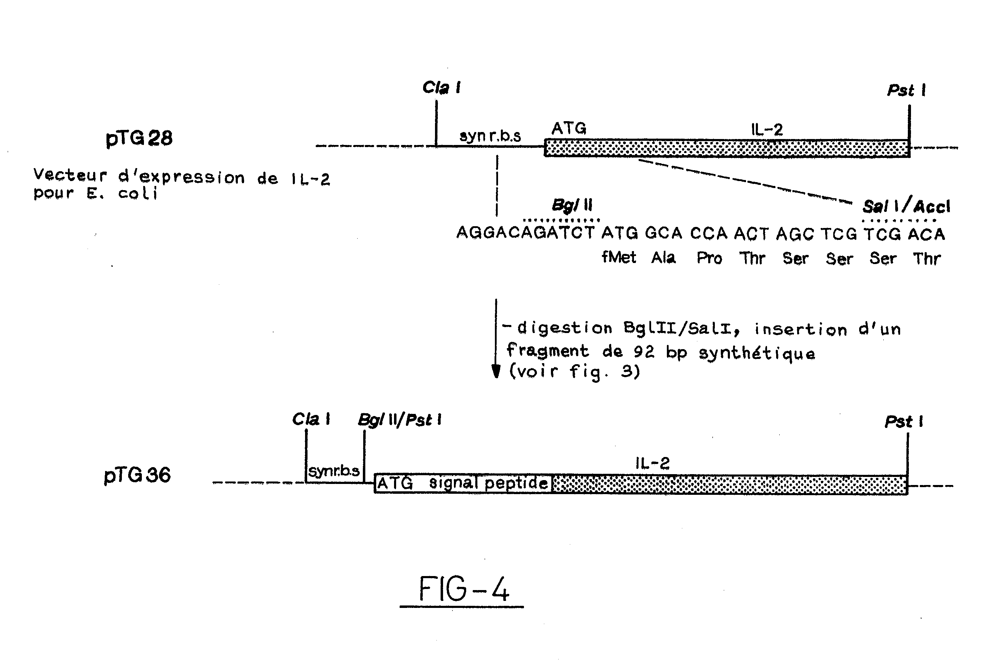

Dans ce but, un site de restriction (Sal I/Acc I) est crée dans le pTG 26 (figure 2). Ensuite le fragment de c-ADN est transféré dans un vecteur d'expression de E. coli ; cette construction intermédiaire est appelée pTG 28 (figure 4). Dans ce pTG 28, on insère un fragment d'ADN synthétique de 92 paires de bases correspondant à la séquence du peptide - signal.

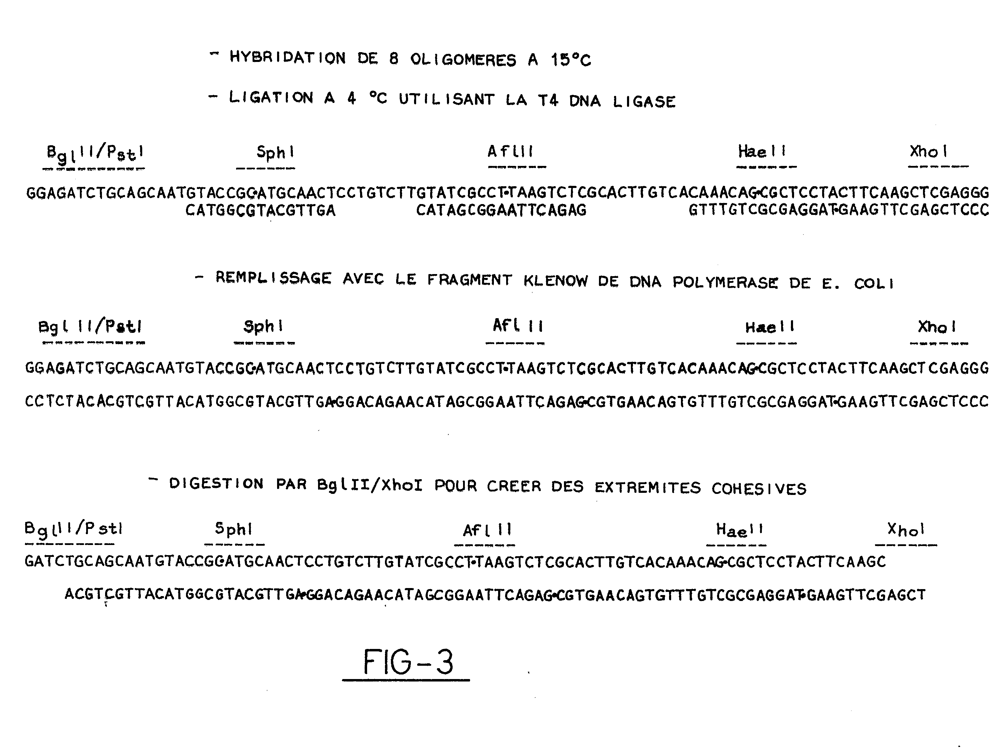

Ce fragment a été préparé par assemblage d'une suite d'oligonucléotides synthétiques (Kohli et al.,1982) utilisant la ligase $T_4$ et l'ADN polymérase (figure 3) . Des terminaisons cohésives ont été générées par digestion avec Bgl II et Xho I. Le fragment a ensuite été inséré entre les sites Bgl II et Sal I présents sur le pTG 28, (figure 4) pour donner le pTG 36.

On confirme par séquençage de l'ADN que le plasmide résultant, pTG 36, possède une structure correcte et qui correspond à celle qui a été déterminée pour le fragment synthétique.

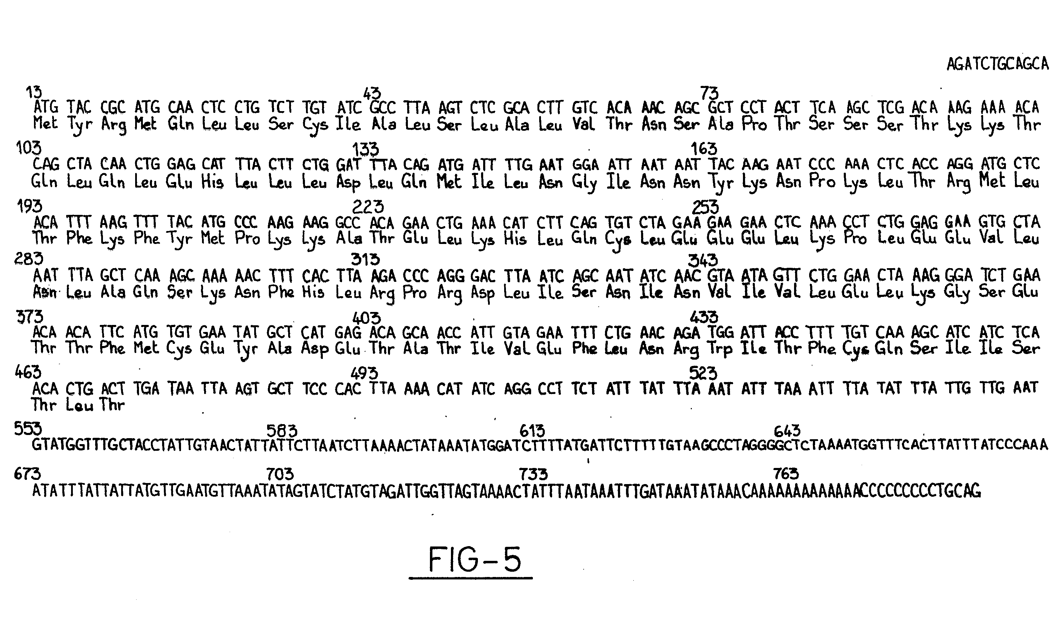

On trouve sur la figure 5 la séquence résultante complète, codant pour l'IL-2, flanquée des sites Bgl II et/ou Pst.

EXEMPLE 2 :

Construction des plasmides hybrides

Les tailles combinées des différents éléments nécessaires pour le transfert de la séquence codant pour l'IL-2 dans le génome de VV et son expression subséquente sont de l'ordre de plusieurs Kb. Il a donc été jugé nécessaire de minimiser la taille du plasmide de réplication dans E.coli utilisé pour le travail de construction de façon à faciliter les manipulations nécessaires.

Le fragment HindIII (Hin-J) du génome de VV contient le gène complet de la thymidine kinase (TK) qui a déjà été utilisé précédemment pour permettre l'échange et la recombinaison de l'ADN inséré dans le génome de VV (Mackett et al., 1982). Il est important de noter que le transfert d'un insert dans le gène TK dans le génome de VV crée un virus TK déficient qui peut être sélectionné. Il a tout d'abord été nécessaire de produire un plasmide de petite taille portant un site unique HindIII utilisable pour l'intégration du fragment Hin-J VV. En outre, il était nécessaire d'éliminer les séquences de restriction non nécessaires du plasmide de façon à permettre les manipulations suivantes.

La construction a été amorcée à partir du plasmide pML2 (Lusky et Botchan, 1981) qui est un vecteur dérivé du plasmide pBR322 par délétion spontanée dans lequel le segment entre les nucléotides 1089 et 2491 a été perdu (figure 6). D'abord la séquence de PstI a été éliminée par insertion du fragment AhaIII-AhaIII de pUC8 (Vieira et Messing, 1982) entre deux sites AhaIII de pML2 en éliminant 19 paires de bases. On a utilisé la méthode de "linker-tailing" (Lathe et al., 1984) pour insérer un linker HindIII entre les sites NruI et EcoRI traité par S1 de ce plasmide, en éliminant le site BamHI. Ceci conduit à un plasmide de 2049 paires de bases portant le gène β-lactamase fonctionnel (conférant la résistance à l'ampicilline ) et comportant en outre une origine de réplication active dans E.coli et un site de restriction unique HindIII.

Cette construction a été appelée pTG1H.

Le fragment Hin-J de l'ADN de VV portant le gène TK a préalablement été cloné dans un vecteur provenant de pBR327 (Drillien et Spehner, 1983). Ce fragment de 4,6 Kb a été recloné dans le site HindIII de pTG1H. Un clone a été sélectionné dans lequel le gène TK est situé distalement par rapport au gène codant pour la résistance à l'ampicilline.

Cette construction pTG1H-TK a été utilisée comme vecteur dans l'expérience suivante.

L'étape suivante a été d'isoler un promoteur de VV utilisable pour commander l'expression de la séquence codant pour l'IL-2 humaine. Le promoteur d'un gène précoce codant pour une protéine de 7500 daltons (7,5 K) a déjà été utilisé avec succès dans un but identique (Smith et al., 1983) et on a donc procédé à l'isolement de ce segment.

Le gène 7,5 K est situé sur l'un des plus petits fragments SalI (fragment Sal-S) du génome de VV type WR (Venkatasan et al., 1981). Comme les petits fragments sont clonés de façon préférentielle, une grande proportion des clones obtenus par clonage direct de l'ADN de VV type WR coupé par SalI dans le plasmide pBR322 porte le fragment Sal-S. Ce fragment est transféré sur le bactériophage vecteur M13mp701 (voir Kieny et al., 1983), par digestion SalI et religation, en conduisant ainsi au phage M13TGSal-S.

Dans ce clone, un site ScaI se trouve immédiatement à proximité de l'ATG d'initiation du gène 7,5 K. En aval du gène 7,5 K se trouvent situés des sites uniques BamHI et EcoRI provenant du vecteur. Les sites BamHI et ScaI sont fusionnés par l'intermédiaire d'un linker BglII 5' -CAGATCTG-3' après avoir complété les extrémités générées par digestion BamHI avec le fragment Klenow de la polymérase de E.coli. Ce procédé élimine le site ScaI mais reconstitue le site BamHI et déplace le site unique EcoRI en aval. En même temps, le site SalI (AccI) en aval est éliminé, le site Sal I en amont devient donc unique.

Cette construction est appelée M13TG 7,5 K.

A l'intérieur du fragment Hind-J de l'ADN de VV se trouvent situés des sites ClaI et EcoRI qui sont séparés par environ 30 paires de bases (Weir et Moss, 1983). Le fragment promoteur de 7,5K présent dans M13TG7,5K est excisé par AccI et EcoRI et cloné entre les sites ClaI et EcoRI de pTG1H-TK pour générer pTG1H-TK-P7,5K dont la synthèse est schématisée figure 7.

Cette construction conduit au transfert des sites BamHI et EcoRI uniques du vecteur M13 immédiatement en aval de la séquence du promoteur 7,5K. Ces sites uniques BamHI et EcoRI sont utilisés dans la construction suivante.

Le segment polylinker du bactériophage M13TG131 (Kieny et al., 1983) est excisé par EcoRI et Bgl II et inséré entre les sites EcoRI et BamHI du plasmide pTG1H-TK-P7,5K, générant pTG186-poly. Dans cette construction, 10 sites de restriction sont disponibles pour le clonage d'un gène étranger sous le contrôle de P7,5K.

pTG186-poly est mis en digestion avec PstI et ligué avec pTG36 digéré par PstI (figure 8).

Après transformation de E.coli, l'un des plasmides recombinants isolé par cette procédure, pVV-IL-2 est sélectionné car il porte le c-ADN de l'IL-2 humaine dans l'orientation correcte pour l'expression à, partir du promoteur P 7,5 K.

pVV-IL-2 sera parfois noté pTG188.

EXEMPLE 3 :
Clonage dans le virus de la vaccine (figure 9)

La stratégie décrite par Smith et al. (1983) repose sur l'échange in vivo entre un plasmide portant un insert dans le gène VV TK et le génome viral de type sauvage de façon à inactiver le gène TK porté par le virus. Les virus TK⁻ peuvent être sélectionnés par étalement sur une lignée cellulaire TK-négative en présence de 5-bromo-déoxyuridine (5BUdR) (Mackett et al. ; 1982). La thymidine kinase phosphoryle le 5BUdR en 5'-monophosphate, qui est ensuite converti en triphosphate. Ce composé est un analogue de dTTP et son incorporation dans l'ADN bloque le développement correct du virus. Un virus TK⁻ peut néanmoins répliquer son ADN normalement et il conduit à des plaques virales visibles dans une monocouche de cellules également TK⁻.

Le virus de la vaccine se propage dans le cytoplasme des cellules infectées plutôt que dans leur noyau. C'est pourquoi il n'est pas possible de tirer avantage de la machinerie de réplication et de transcription de l'ADN de la cellule hôte et il est nécessaire que le virion possède ses propres composants pour l'expression du génome viral. L'ADN de VV purifié est non-infectieux.

Afin de générer les recombinants, il est nécessaires d'effectuer simultanément l'infection cellulaire avec un VV et une transfection avec le segment d'ADN cloné qui présente de l'intérêt. Toutefois, la génération des recombinants est limitée à une petite proportion des cellules compétentes pour la transfection à l'ADN. C'est pour cette raison qu'il a été nécessaire de mettre en oeuvre une stratégie de "congruence" indirecte pour réduire le bruit de fond des virus parentaux non-recombinants. Ceci a été effectué en utilisant comme virus infectieux vivant un mutant thermosensible (ts) de la vaccine qui n'est pas capable de se propager à une température non permissive de 39,5° C (Drillien et Spehner, 1983). Lorsque les cellules sont infectées avec un mutant ts dans des conditions non permissives et transfectées avec l'ADN d'un virus de type sauvage, la multiplication virale interviendra seulement dans les cellules qui sont compétentes pour la transfection et dans lesquelles une recombinaison entre l'ADN viral sauvage et le génome du virus ts aura eu lieu ; aucun résultat ne sera obtenu dans les autres cellules, en dépit du fait qu'elles ont été infectées. Si un plasmide recombinant contenant un fragment de l'ADN de vaccine tel que pVV-IL-2 est inclus dans le mélange de transfection, à la concentration appropriée, avec l'ADN du virus de type sauvage, il est également possible d'obtenir qu'il participe à la recombinaison homologue avec l'ADN de la vaccine, dans les cellules compétentes.

Des monocouches de cellules primaires de fibroblastes d'embryons de poulets (CEF) sont infectées à 33° C avec VV-Copenhague ts7 (0,1 pfu/cellule) et transfectées avec un coprécipité au phosphate de calcium de l'ADN du virus de type sauvage VV-Copenhague (50 ng/$10^6$ cellules) et du plasmide recombinant (25-30 ng/$10^6$ cellules).

Après incubation pendant 2 heures à une température qui ne permet pas le développement du virus ts (39,5° C), les cellules sont rincées et incubées pendant 48 heures à 39,5°C. Des dilutions de virus $ts^+$ sont utilisées pour réinfecter une monocouche de cellules de souris L-TK$^-$ qui sont incubées à 37° C en présence de 5BUdR (100 µg/ml). Différentes plaques TK$^-$ sont obtenues à partir des cellules qui ont reçu le plasmide recombinant, tandis que les cultures de contrôle sans plasmide ne montrent pas de plaques visibles. Les virus TK$^-$ sont ensuite sous-clonés par une 2ème sélection en présence de 5BUdR.

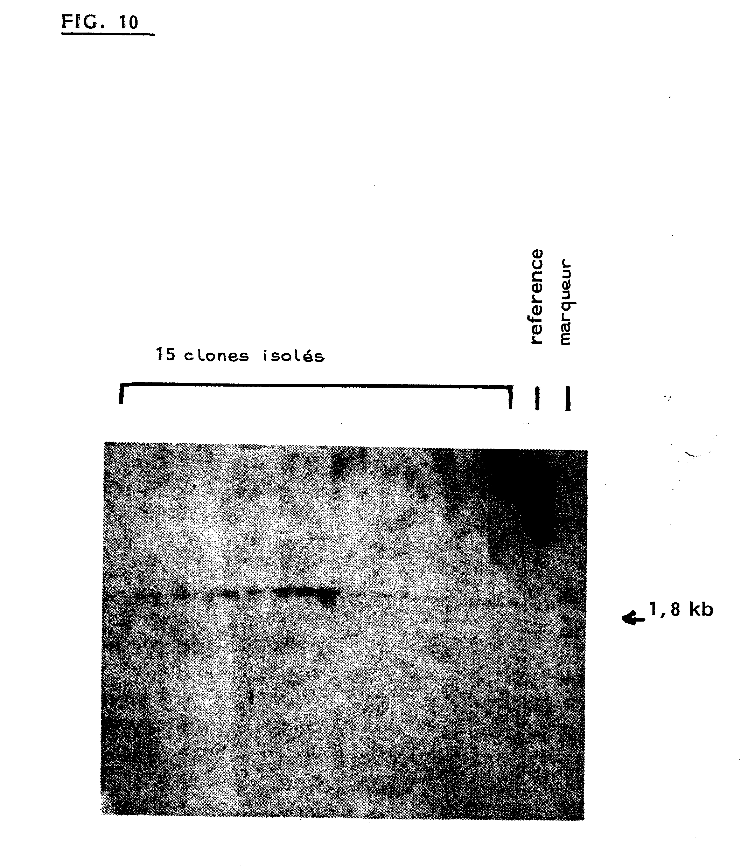

Une double recombinaison réciproque entre le plasmide hybride IL-2/vaccine et le génome de VV permet d'échanger le gène viral TK avec le gène TK portant l'insert IL-2 présent dans le plasmide. Dans le génome de VV le gène TK est présent sur un fragment unique HindIII : Hin-J. Les recombinants ayant transféré le bloc d'expression de l'IL-2 sont sensés avoir intégré un site interne HindIII dérivant du polylinker. C'est pourquoi les ADN purifiés à partir du virus TK⁻ sont digérés avec HindIII et soumis à une électrophorèse sur gel d'agarose. Les fragments d'ADN sont alors transférés sur un filtre de nitro-cellulose selon la technique décrite par Southern (1975). Le filtre est ensuite hybridé avec le fragment PstI-PstI de 0,76 Kb contenant la séquence IL-2 marquée au $^{32}$P par "nick translation". Après lavage du filtre, ce dernier est fluorographié et une bande de 1,8 Kb est visible sur l'autoradiographie quand le virus de la vaccine a incorporé le gène IL-2 (figure 10).

Les virus recombinants sont alors analysés pour leur capacité d'exprimer de l'IL-2 dans les surnageants de cellules infectées.

EXEMPLE 4 :

Expression de l'IL-2 humaine à partir des virus de vaccine recombinants

Des mono-couches de cellules BHK (Baby Hamster Kidney) confluentes sont infectées avec VV-IL-2 (0,1 pfu/cellule) pendant 1 heure à température ambiante puis un milieu de culture est ajouté et l'incubation est poursuivie à 37° C pendant 24 heures. Les surnageants sont alors récoltés, le virus est inactivé par un traitement au β-propiolactone (1/4000 e ; 24 h à 4° C puis 2 h à 37° C).

L'activité IL-2 est ensuite dosée dans les surnageants selon une technique décrite par Gilles et al. (1978).

EXEMPLE 5 :

Dosage biologique de l'IL-2

a) principe

Dans un test biologique sont comparées les mesures de la prolifération de cellules IL-2 dépendantes en présence de deux surnageants standard définissant l'unité IL-2 et son double, et du surnageant à doser. L'IL-2 humaine est active chez la souris, c'est la raison pour laquelle on a utilisé la lignée murine CTLL-2 comme cellules IL-2 dépendantes.

b) Réalisation

Dans des plaques de culture à 96 puits à fond rond (GREINER) les surnageants standards et les surnageants à doser sont dilués de 1/2 en 1/2 (5 dilutions) dans du RPMI seul, chaque dilution est faite en double. Ensuite dans chaque puits où se trouve déjà 60 μl de dilution du surnageant à doser, sont ajoutés dans 60 μl de milieu (RPMI 1640 + 20 % SVF + 2 mercapto éthanol $10^{-4}$M) $4.10^3$ CTLL$_2$. Après 48 h de culture à l'incubateur, un test de prolifération cellulaire est pratiqué sur ces cellules par l'incorporation de $[^3H]$- déoxythymidine.

c) Calcul des unités

L'activité IL-2 se définit en unité/ml. c'est la capacité qu'une solution à tester a de permettre la prolifération de cellules tests dépendantes de l'IL-2, par rapport à une solution standard contenant par définition 1 unité IL-2/ml, ainsi l'activité IL-2 d'un surnageant est déduite de l'observation des courbes cpm/dilution, obtenues avec le surnageant de référence et les surnageants à tester. La "solution standard interne" utilisée correspond à 50 unités internationales IRP (international reference preparation) comme définies lors d'un congrès de standardisation des lymphokines (Dumonde et Papermaster, 1984). Pour tout dosage d'un surnageant est définie une courbe cpm/dilution, cette courbe est généralement de type sigmoïdal. La détermination de l'activité IL-2 est effectuée sur la partie linéaire de ces courbes. Le calcul montre qu'on est toujours bien placé lorsqu'on compare les différentes courbes au niveau de leur intersection avec une droite parallèle à l'abscisse et située sur l'ordonnée au 1/3 de la valeur maximum des cpm obtenues avec l'IL-2 standard. Un programme fonctionnant sur un Apple II effectue ce calcul au laboratoire.

d) Résultats

Après 24 heures d'incubation, les surnageants des cellules BHK infectées par une série de 16 virus recombinants de la vaccine IL-2, isolés indépendamment, ont été dilués dans 10 fois leur volume de PBS, et testés pour l'activité IL-2.

Une valeur moyenne de 165 u/ml de milieu de culture a été trouvée avec un maximum de 300 u/ml.

Les surnageants des cellules de contrôle infectées par le virus de la vaccine de type sauvage n'ont montré aucune activité IL-2.

Le plasmide pTG 188 a été déposé le 20 juin 1985 à la Collection Nationale de Cultures de Microorganismes de l'Institut Pasteur, 28 rue du Docteur Roux, 75015 PARIS, sous le numéro suivant : .E. coli souche : 5K pTG 188 : n° I-458.

La description de ce plasmide est la suivante :

Plasmide de réplication de E. coli portant un c-ADN de l'interleukine 2 humaine (IL-2) placé sous le contrôle d'un promoteur du virus de la vaccine et destiné à la recombinaison _in vivo_, dans les cellules de mammifères, avec le virus de la vaccine, pour engendrer un virus recombinant vaccine-TK-IL-2, capable d'exprimer l'IL-2 dans des cellules de mammifères.

. Le c-ADN de l'IL-2 a été isolé d'une banque provenant d'ARN messager de lymphocytes stimulés par un agent mitogène.

. Ce c-ADN a été placé sous le contrôle d'un promoteur du virus de la vaccine, le promoteur de la protéine 7,5 K.

. La séquence d'ADN P 7,5 K - IL-2 a été intégrée dans le gène TK de la vaccine pour favoriser une recombinaison homologue avec le gène TK du virus de la vaccine de type sauvage qui sera utilisé pour infecter les cellules de mammifères.

. La séquence d'ADN comprenant P 7,5 K - IL-2 intégrée dans le gène TK a été insérée dans un plasmide de E. coli dérivé du pML2 et comprenant une origine de réplication dans E. coli et le gène de la β lactamase.
Cette construction s'appelle pTG188.

L'ADN du plasmide pTG188 sera utilisé sous forme de précipité de phosphate de calcium pour transfecter des cellules de mammifères infectées par le virus de la vaccine et obtenir ainsi, au cours de la multiplication virale dans la cellule, une recombinaison entre le gène TK du virus et le gène TK contenant le gène IL-2 porté par le plasmide. Le virus recombinant résultant de cet échange sera TK⁻ et portera le gène de l'IL-2.

17

## REFERENCES

Drillien, R. & Spehner, D. (1983) Virology 131, 385-393.

Dumonde, D.C & Papermaster, B.W. (1984) Lymphokine Res. 3, 193.

Gillis, S.,Fern, M.M., Ou, W. & Smith, K.A. (1979)Exp.Med. 149, 1460.

Hansen, J.A., Martin, P.J. & Nowinski, R.C. (1980) Immuno-genetics 10, 247.

Hefenerder, S.H., Carlan, P.J., Henney, C.S. & Gillis, J. (1983) J. Immunol. 130,222

Kieny, M.P., Lathe, R. & Lecocq, J.P. (1983) Gene 26, 91-99.

Kieny, M.P., Lathe, R., Drillien R., Spehner A., Skory S., Schmitt D., Wiktor T., Koprowski H. & Lecocq J.P. (1984) Nature, 312, 163-166.

Kohli, V., Balland, A., Wintzerith, M., Sauerwald, R., Staub, A. & Lecocq, J.P. (1982) Nucleic Acids Res. 10, 7439-7448.

Lathe, R., Kieny, M.P., Skory, S. & Lecocq, J.P. (1984) DNA, in press.

Lusky, M., Botchan, M.(1981) Nature 293, 79-81.

Mackett, M., Smith, J.L. & Moss, B. (1982) Proc. Natl. Acad. Sci. USA 79, 7415-7419.

Morgan, D.A., Ruscetti, R.C. & Gallo, R.C. (1976) Sciene 193, 1007.

Panicali , D. & Paoletti, E. (1982), Proc. Natl. Acad. Sci. USA 79, 4927-4931.

Panicali, D., Davis, S.W., Weinberg, R.L. & Paoletti, E. (1983) Proc. Natl. Acad. Sci. USA 80, 5364-5368.

Smith, G.L., Mackette- M. & Moss, V. (1983) Nature 302, 490-495.

Soberon et coll., Gene 9, 287-305 (1980).

Southern, E.M. (1975) J. Molec. Biol. 98 , 503.

Taniguchi, T., Matsui, H., Fujita, T., Takaoka,C., Kashima, N., Yoshimoto, R & Hamuro, J. (1983) Nature 302, 305.

Venkatesan, S., Baroudy, B.M. & Moss, B. (1981) Cell 125, 805-813.

Vieira, J. & Messing, J. (1982) Gene 19, 259-268.

Watson, J. & Mochiguki, D. (1980) Immunol. Rev. 51, 257.

Weir, J.P. & Moss, B. 1983 J. Virol. 46, 530-537.

## REVENDICATIONS

1. Poxvirus caractérisé en ce qu'il comporte tout ou partie de la séquence d'ADN codant pour une protéine IL-2 humaine.

2. Poxvirus selon la revendication 1, caractérisé en ce qu'il s'agit d'un virus de la vaccine.

3. Poxvirus selon les revendications 1 et 2, caractérisé en ce que la séquence d'ADN codant pour une IL-2 humaine est une séquence d'ADN codant pour la protéine mature complète.

4. Poxvirus selon les revendications 1 à 3, caractérisé en ce que la séquence d'ADN codant pour l'IL-2 humaine est sous la dépendance d'un promoteur d'un gène du virus utilisé.

5. Poxvirus selon la revendication 4, caractérisé en ce que le promoteur est le promoteur du gène de la protéine 7,5 K du virus de la vaccine.

6. Poxvirus selon l'une des revendications 1 à 5, caractérisé en ce que la séquence d'ADN codant pour l'IL-2 humaine est clonée à l'intérieur d'un gène non essentiel, du virus.

7. Poxvirus selon la revendication 6, caractérisé en ce que le gène non essentiel est le gène TK.

8. Poxvirus selon l'une des revendications 1 à 7, caractérisé en ce qu'il comporte en outre une séquence codant pour un antigène vaccinal.

9. Cellule de mammifère infectée par un virus selon l'une des revendications 1 à 8.

10. Procédé de préparation d'une protéine IL-2 humaine caractérisé en ce que l'on cultive des cellules selon la revendication 9 et que l'on récupère la protéine formée dans le milieu de culture.

11. Protéine IL-2 humaine obtenue par la mise en oeuvre du procédé selon la revendication 10.

12. Composition pharmaceutique caractérisée en ce qu'elle contient à titre de principe actif, curatif ou préventif, une IL-2 humaine selon la revendication 11.

13. Composition pharmaceutique, utile notamment à la génération d'IL-2 in vivo chez l'homme ou l'animal, caractérisée en ce qu'elle comporte un poxvirus selon l'une des revendications 1 à 8 et un support pharmaceutiquement acceptable.

14. Composition pharmaceutique selon la revendication 12, caractérisée en ce que le poxvirus utilisé est vivant.

15. Composition pharmaceutique selon les revendications 13 et 14, caractérisée en ce qu'elle comporte un support pharmaceutiquement acceptable permettant son administration par voie injectable.

16. Composition pharmaceutique selon la revendication 13, caractérisée en ce qu'elle comporte en outre un antigène vaccinal.

```
                                        G
                                        ↑
10                               40     .
ACA AAG AAA ACA CAG CTA CAA CTG GAG CAT TTA CTT CTG GAT TTA CAG ATG ATT TTG AAT GGA ATT AAT AAT TAC AAG AAT CCC AAA CTC
Thr Lys Lys Thr Gln Leu Gln Leu Glu His Leu Leu Leu Asp Leu Gln Met Ile Leu Asn Gly Ile Asn Asn Tyr Lys Asn Pro Lys Leu

100                              130                              160
ACC AGG ATG CTC ACA TTT AAG TTT TAC ATG CCC AAG AAG GCC ACA GAA CTG AAA CAT CTT CAG TGT CTA GAA GAA GAA CTC AAA CCT CTG
Thr Arg Met Leu Thr Phe Lys Phe Tyr Met Pro Lys Lys Ala Thr Glu Leu Lys His Leu Gln Cys Leu Glu Glu Glu Leu Lys Pro Leu

190                              220                              250
GAG GAA GTG CTA AAT TTA GCT CAA AGC AAA AAC TTT CAC TTA AGA CCC AGG GAC TTA ATC AGC AAT ATC AAC GTA ATA GTT CTG GAA CTA
Glu Glu Val Leu Asn Leu Ala Gln Ser Lys Asn Phe His Leu Arg Pro Arg Asp Leu Ile Ser Asn Ile Asn Val Ile Val Leu Glu Leu

280                              310                              340
AAG GGA TCT GAA ACA ACA TTC ATG TGT GAA TAT GCT GAT GAG ACA GCA ACC ATT GTA GAA TTT CTG AAC AGA TGG ATT ACC TTT TGT CAA
Lys Gly Ser Glu Thr Thr Phe Met Cys Glu Tyr Ala Asp Glu Thr Ala Thr Ile Val Glu Phe Leu Asn Arg Trp Ile Thr Phe Cys Gln
                        A
                        ↑
370                              400                              430
AGC ATC ATC TCA ACA CTG ACT TGA TAA TTA AGT GCT TCC CAC TTA AAA CAT ATC AGG CCT TCT ATT TAT TTA AAT ATT TAA ATT TTA TAT
Ser Ile Ile Ser Thr Leu Thr xxx

460                    490                         520                          550
TTATTGTTGAATGTATGGTTTGCTACCTATTGTAACTATTATTCTTAATCTTAAAACTATAAATATGGATCTTTTATGATTCTTTTTGTAAGCCCTAGGGGCTCTAAAATGGTTTCACTT
580                    610                         640                          670
ATTTATCCCAAAATATTTATTATTATGTTGAATGTTAAATATAGTATCTATGTAGATTGGTTAGTAAAACTATTTAATAAATTTGATAAATATAAACAAAAAAAAAAAAAAACCCCCCCCC
```

1/10

0206939

FIG-1

Thr Lys Lys Thr
(pBR 322-Ap)— — —CTGCAGG(G)₇GGG GGG.ACA.AAG.AAA.ACA.— — — — — — — — (IL-2 cont.)

\*\*
GGGGG TCG ACA AAG A                    synthétique 15 − mer

MUTATION DE DEUX BASES PAR UN 15-MER
QUI EST HYBRIDE AVEC LE BRIN SIMPLE
D'UN DERIVE DE M13 CONTENANT UN INSERT
DU pTG26 INCORPORANT IL-2 cDNA

Ser Thr Lys Lys Thr
— — — — — — —GGGGG.TCG.ACA.AAG.AAA.ACA.— — — — — — — — (IL-2 cont.)
Sal I/Acc I

# FIG-2

0206939

2 / 10

- HYBRIDATION DE 8 OLIGOMERES A 15°C

- LIGATION A 4 °C UTILISANT LA T4 DNA LIGASE

Bgl II/Pst I        SphI        AflII        HaeII        XhoI

GGAGATCTGCAGCAATGTACCGGATGCAACTCCTGTCTTGTATCGCCTTAAGTCTCGCACTTGTCACAAACAGCGCTCCTACTTCAAGCTCGAGGG
        CATGGCGTACGTTGA        CATAGCGGAATTCAGAG        GTTTGTCGCGAGGATGAAGTTCGAGCTCCC

- REMPLISSAGE AVEC LE FRAGMENT KLENOW DE DNA POLYMERASE DE E. COLI

Bgl II/PstI        SphI        AflII        HaeII        XhoI

GGAGATCTGCAGCAATGTACCGGATGCAACTCCTGTCTTGTATCGCCTTAAGTCTCGCACTTGTCACAAACAGCGCTCCTACTTCAAGCTCGAGGG

CCTCTACACGTCGTTACATGGCGTACGTTGAGGACAGAACATAGCGGAATTCAGAGCGTGAACAGTGTTTGTCGCGAGGATGAAGTTCGAGCTCCC

- DIGESTION PAR BglII/XhoI POUR CREER DES EXTREMITES COHESIVES

Bgl II/PstI        SphI        AflII        HaeII        XhoI

GATCTGCAGCAATGTACCGGATGCAACTCCTGTCTTGTATCGCCTTAAGTCTCGCACTTGTCACAAACAGCGCTCCTACTTCAAGC

  ACGTCGTTACATGGCGTACGTTGAGGACAGAACATAGCGGAATTCAGAGCGTGAACAGTGTTTGTCGCGAGGATGAAGTTCGAGCT

## FIG-3

0206939

pTG 28

Vecteur d'expression de IL-2
pour E. coli

Cla I      Pst I

synr.b.s   ATG   IL-2

Bgl II      Sal I/Accl

AGGACAGATCT ATG GCA CCA ACT AGC TCG TCG ACA

fMet Ala Pro Thr Ser Ser Ser Thr

- digestion BglII/SalI, insertion d'un
fragment de 92 bp synthétique
(voir fig. 3)

Cla I   Bgl II/Pst I      Pst I

pTG 36

synr.b.s   ATG   signal peptide   IL-2

FIG-4

```
AGATCTGCAGCA

13                                         43                                        73
ATG TAC CGC ATG CAA CTC CTG TCT TGT ATC    GCC CTT GTC ACA AAC AGC CCT ACT TCA AGC    TCG ACA AAG AAA ACA
Met Tyr Arg Met Gln Leu Leu Ser Cys Ile    Ala Leu Val Thr Asn Ser Ala Pro Thr Ser    Ser Thr Lys Lys Thr

103                                        133                                       163
CAG CTA CAA CTG GAG CAT TTA CTT CTG GAT    TTA CAG ATT TTG AAT GGA ATG ATT CTC AAC    AAA CCC AAT TAC AGG ATG CTC
Gln Leu Gln Leu Glu His Leu Leu Leu Asp    Leu Gln Ile Leu Asn Gly Met Ile Leu Asn    Lys Pro Asn Tyr Arg Met Leu

193                                        223                                       253
ACA TTT AAG TTT TAC ATG CCC AAG AAG GCC    ACA GAA GAA CTC AAA CAT CTT CAG TGT CTA    GAA GAA CTG GTG CTA
Thr Phe Lys Phe Tyr Met Pro Lys Lys Ala    Thr Glu Glu Leu Lys His Leu Gln Cys Leu    Glu Glu Leu Val Leu

283                                        315                                       343
AAT TTA GCT CAA AGC AAA GAG AGC AAC TTT    CAC AGA CCC AGG GAC GAT ATA GTT AAC ATC    AGC AAT ATC CTG GAA CTA AAG
Asn Leu Ala Gln Ser Lys Glu Ser Asn Phe    His Arg Pro Arg Asp Asp Ile Val Asn Ile    Ser Asn Ile Leu Glu Leu Lys

373                                        403                                       433
ACA ACA TTC ATG TGT GAA TAT GCT CAT GAG    GCA ACA GCA ACC ATT GTA GAA TTT CTG AAC    AGA ATT ACC TTT TGT CAA AGC ATC TCA
Thr Thr Phe Met Cys Glu Tyr Ala His Glu    Ala Thr Ala Thr Ile Val Glu Phe Leu Asn    Arg Ile Thr Phe Cys Gln Ser Ile Ser

463                                        493                                       523
ACA CTG ACT TGA TAA TTA AGT GCT TTA AAA    CAT ATC AGG CCT TCT ATT TAT TTA AAT ATT    TAA ATT TTA TTG AAT
Thr Leu Thr

553                                        583                             613                 643
GTATGGGTTGCTACCTATTGTAACTATTATTCTTAATCTTAAAACTATAAATATGGATCTTTTGTGAAGCCCTAGGGGCTCTAAAATGGTTTCACTTATTATTTATCCCAAA

673                                        703                             735                 765
ATATTTATTATTATGTTGAATGTTAAATATAGTATCTATGTGTAGATTGGTTAGTAAAAACTATTTAATAAATTTGATAAATATAAACAAAAAAAAAACCCCCCCCTGCAG
```

FIG-5

0206939

FIG-6

FIG-7

0206939

FIG-8

FIG-9

FIG. 10

15 clones isolés

reference

marqueur

← 1,8 kb

Office européen
des brevets

**RAPPORT DE RECHERCHE EUROPEENNE**

0206939

Numéro de la demande

EP    86 40 1349

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | NATURE, vol. 312, no. 5990, novembre 1984, pages 163-166, Reading, Berks, GB; M.P. KIENY et al.: "Expression of rabies virus glycoprotein from a recombinant vaccinia virus" * En entier * --- | 1-10, 13-16 | C 12 N   15/00 C 12 P   21/02 A 61 K   37/02 A 61 K   35/76 C 12 N    5/00 C 12 N    7/00 |
| Y | EP-A-0 110 385  (THE UNITED STATES OF AMERICA) * En entier * --- | 1-10, 13-16 | |
| Y | JOURNAL OF VIROLOGY, vol. 49, no. 3, mars 1984, pages 857-864, American Society for Microbiology; M. MACKETT et al.: "General method for production and selection of infectious vaccinia virus recombinants expressing foreign genes" * En entier * --- | 1-10, 13-16 | **DOMAINES TECHNIQUES RECHERCHES (Int. Cl.4)** C 12 N C 12 P A 61 K |
| Y | PROCEEDINGS OF THE NATIONAL ACADEMY OF SCIENCES OF THE USA, vol. 80, no. 23, décembre 1983, pages 7155-7159, Washington, US; G.L. SMITH et al.: "Construction and characterization of an infectious vaccinia virus recombinant that expresses the influenza hemagglutinin gene and induces resistance to influenza virus infection in hamsters" * En entier * ---                    -/- | 1-10, 13-16 | |

Le présent rapport de recherche a été établi pour toutes les revendications

| Lieu de la recherche | Date d'achèvement de la recherche | Examinateur |
|---|---|---|
| LA HAYE | 08-09-1986 | DESCAMPS J.A. |

0206939

**RAPPORT DE RECHERCHE EUROPEENNE**

Numéro de la demande

Office européen
des brevets

EP 86 40 1349

| | DOCUMENTS CONSIDERES COMME PERTINENTS | | Page 2 |
|---|---|---|---|

| Catégorie | Citation du document avec indication, en cas de besoin, des parties pertinentes | Revendication concernée | CLASSEMENT DE LA DEMANDE (Int. Cl.4) |
|---|---|---|---|
| Y | SCIENCE, vol. 224, 27 avril 1984, pages 397-399; G.L. SMITH et al.: "Plasmodium knowlesi sporozoite antigen: Expression by infectious recombinant vaccinia virus" * En entier * --- | 1-10, 13-16 | |
| Y | GENE, vol. 35, no. 1/2, 1985, pages 169-177, Elsevier Science Publishers, Amsterdam, NL; D.B. BOYLE et al.: "Multiple-cloning-site plasmids for the rapid construction of recombinant poxviruses" * En entier * --- | 1-10, 13-16 | |
| X | EP-A-0 091 539 (JAPANESE FOUNDATION FOR CANCER RESEARCH) * En entier * | 11,12 | |
| Y | --- ----- | 1-10, 13-16 | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 08-09-1986 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82